# EUROPEAN PATENT SPECIFICATION

(11) **EP 2 664 606 B1**
(45) Date of publication and mention of the grant of the patent: **14.09.2016**
(21) Application number: 13164848.7
(22) Date of filing: 23.04.2013
(51) Int. Cl.: A23L 29/30

(54) **An inhibitor of the production of advanced glycation end products**
Hemmer der Produktion von fortgeschrittenen Glykierungsendprodukten
Inhibiteur de la production de produits terminaux de glycation avancée

(30) Priority: 25.04.2012 JP 2012100287; 30.11.2012 JP 2012263311; 30.11.2012 JP 2012263314
(43) Date of publication of application: 20.11.2013
(73) Proprietor: Ueno Fine Chemicals Industry, Ltd., Osaka-shi, Osaka-fu (JP)
(72) Inventor: Yamamoto, Hiroshi, Kanazawa-shi, Ishikawa (JP); Munesue, Seiichi, Kanazawa-shi, Ishikawa (JP); Honda, Junya, Osaka-shi, Osaka (JP); Kawaura, Ryosuke, Osaka-shi, Osaka (JP); Kuretani, Aya, Osaka-shi, Osaka (JP)
(74) Representative: Beckmann, Claus

(56) References cited:
- WO-A2-2009/072817
- CA-A1- 2 455 805
- KR-A- 20030 018 730

## Description

### TECHNICAL FIELD

The present invention relates to an inhibitor of the production of advanced glycation end products, which contains a sugar alcohol as an active ingredient, and a medicament for the prevention and/or treatment of diabetes or diabetic complications, which contains a sugar alcohol as an active ingredient.

### BACKGROUND ART

A phenomenon of browning, which occurs when a mixture of amino acids and reducing sugars is heated, is generally called Maillard reaction and, in food field, is known as a phenomenon which occurs during a heat treatment or storage of food. Maillard reaction also occurs in vivo. Since glycosylated hemoglobin (HbAlc) was identified in vivo in 1968, it was revealed that glycation reaction of proteins proceeds with progression of diabetes or aging. In recent years, it has been reported that advanced glycation end products (hereinafter also referred to as "AGEs") in the glycation reaction of proteins are involved in the development of lifestyle diseases such as diabetic complications and arteriosclerosis and in the progression of aging (Non-Patent Documents 1 to 3). AGEs do not refer to specific substances and the whole picture thereof has not been yet clarified, but in terms of fluorescence or the presence or absence of cross-linked structure, the existence of various substances such as pentosidine, pyrropyridine, and pyrraline has been confirmed.

Details of glycation reaction of proteins in vivo have not been clarified. Amino groups in proteins react with aldehyde groups in reducing sugars to form Schiff bases, leading to the production of stable Amadori compounds, after further long-term reaction, various AGEs are formed from the Amadori compounds. It is known that in the process of AGEs production from the Amadori compounds, dicarbonyl compounds such as 3-deoxyglucosone (hereinafter also referred to as "3-DG"), glyoxal, and methylglyoxal are formed as intermediates. Among these intermediates, 3-DG is considered as an important intermediate because it is extremely highly reactive with amino groups and is most abundantly formed. There is a report that higher serum 3-DG levels are associated with more rapid progression of complications in diabetic patients (Non-Patent Document 4). Therefore, particular attention is directed to the inhibition of 3-DG production to inhibit the production of AGEs.

Maillard reaction inhibitors containing compounds such as aminoguanidine that inhibit late-stage glycosylation of target proteins by reacting with carbonyl groups in early-stage glycosylation products such as 3-DG are disclosed (Patent Document 1). Aminoguanidine is a substance that has been most studied in the development of medicaments in the relevant technical field, but is confirmed to cause adverse drug reactions such as hepatic impairment, thus it is not yet in practical use.

In addition, Maillard reaction inhibitors that inhibit 3-DG producing reaction by extracts from plants such as Uncaria gambir and white birch (Patent Document 2), carbonyl stress-ameliorating agents through the removal of 3-DG, containing enzymes with glyoxalase I activity and carbonyl compound-reducing agents as active ingredients (Patent Document 3), 3-DG production inhibitors containing parabanic acid derivatives as active ingredients (Patent Document 4), and deoxyglucosone production inhibitors containing sulfide compounds as active ingredients (Patent Document 5) are also known.

Patent documents 6 and 7 disclose compositions comprising sugar alcohols, without disclosing any effect on the production of 3-DG or AGE.

In these circumstances, highly safe drugs that can be used to inhibit the production of AGEs involving in the development or exacerbation of various diseases and that cause no serious adverse drug reactions are needed.

### Prior Art Documents:

### Patent Document:

[Patent Document 1] JP 06-67827 B
[Patent Document 2] JP 4195840 B1
[Patent Document 3] JP 4812996 B1
[Patent Document 4] JP 10-182460 A
[Patent Document 5] JP 2007-261983 A
[Patent Document 6] WO2009/07281 A2
[Patent Document 7] KR2003-0018730

### Non-Patent Document:

[Non-Patent Document 1] The Journal of Clinical Investigation, 1993, vol. 91, pp.2470-2478
[Non-Patent Document 2] The New England Journal of Medicine, 1991, vol. 325, pp.836-842
[Non-Patent Document 3] The Biochemical Journal, 2000, vol. 350, pp.381-387
[Non-Patent Document 4] Diabetes Care, 2003, vol. 26, pp.1889-1894

### SUMMARY OF THE INVENTION

An object of the present invention is to provide an AGEs production inhibitor. Another object of the present invention is to provide a medicament which is useful for the prevention and/or treatment of lifestyle diseases, particularly diabetes or diabetic complications, and which causes less adverse drug reactions.

The present inventors have found that one or more sugar alcohols selected from maltitol and sorbitol have the effect to inhibit the production of AGEs and that of 3-DG which is an intermediate of AGEs producing reaction, and then completed the present invention.

The present invention provides an inhibitor of the production of advanced glycation end products, which contains, as active ingredients, one or more sugar alcohols selected from maltitol and sorbitol (hereinafter also referred to as an "AGEs production inhibitor of the present invention").

The present invention also provides a 3-deoxyglucosone production inhibitor containing, as active ingredients, one or more sugar alcohols selected from maltitol and sorbitol (hereinafter also referred to as a "3-DG production inhibitor of the present invention").

The present invention also provides a medicament for the prevention and/or treatment of diabetes or diabetic complications, containing, as active ingredients, one or more sugar alcohols selected from maltitol and sorbitol (hereinafter also referred to as a "medicament for the prevention/treatment of diabetes of the present invention").

The present invention also provides a medicament for non-human animals, for the prevention and/or treatment of diabetes or diabetic complications in non-human animals, containing one or more sugar alcohols selected from maltitol and sorbitol; a method of preventing and/or treating diabetes or diabetic complications in non-human animals, which comprises administering the medicament, or an AGEs production inhibitor or a 3-DG production inhibitor of the present invention to non-human animals; a method of producing functional food or drink, which comprises mixing or adding an AGEs production inhibitor or a 3-DG production inhibitor of the present invention to a food or drink; and use of an AGEs production inhibitor or 3-DG production inhibitor of the present invention for the production of supplements.

The present invention also provides a method of preventing and/or treating diabetes or diabetic complications, which comprises administering an effective amount of one or more sugar alcohols selected from maltitol and sorbitol to a subject in need of the prevention and/or treatment of diabetes or diabetic complications (hereinafter also referred to as a "method of preventing/treating of diabetes of the present invention"); and a method of inhibiting the production of 3-deoxyglucosone or an advanced glycation end product using one or more sugar alcohols selected from maltitol and sorbitol.

The present invention also provides one or more sugar alcohols selected from maltitol and sorbitol for use in the prevention and/or treatment of diabetes or diabetic complications; and one or more sugar alcohols selected from maltitol and sorbitol for use in a method of inhibiting the production of 3-deoxyglucosone or an advanced glycation end product.

The present invention also provides use of one or more sugar alcohols selected from maltitol and sorbitol as an inhibitor of the production of 3-deoxyglucosone or an advanced glycation end product.

The present invention also provides use of one or more sugar alcohols selected from maltitol and sorbitol in the manufacture of a medicament for the prevention and/or treatment of diabetes or diabetic complications.

The present invention also provides use of one or more sugar alcohols selected from maltitol and sorbitol in the manufacture of an inhibitor of the production of 3-deoxyglucosone or an advanced glycation end product; and use of one or more sugar alcohols selected from maltitol and sorbitol as an inhibitor of the production of 3-deoxyglucosone or an advanced glycation end product.

The present invention also provides one or more sugar alcohols selected from maltitol and sorbitol for use in the manufacture of a medicament for the prevention and/or treatment of diabetes or diabetic complications; and one or more sugar alcohols selected from maltitol and sorbitol for use in the manufacture of an inhibitor of the production of 3-deoxyglucosone or an advanced glycation end product.

AGEs, and 3-DG which is a main intermediate in the AGEs producing reaction, are implicated in various lifestyle diseases. Particularly, AGEs and 3-DG are deeply involved in the development and/or progression of diabetes and its complications. Therefore, maltitol and sorbitol, each having been found by the present inventors to have the effect to inhibit the production of AGEs and 3-DG, are useful as active ingredients of a medicament for the prevention and/or treatment of diabetes or diabetic complications, which causes less adverse drug reactions.

### DETAILED DESCRIPTION OF THE INVENTION

One or more sugar alcohols selected from maltitol and sorbitol, which are active ingredients of an AGEs production inhibitor, a 3-DG production inhibitor and a medicament for the prevention and/or treatment of diabetes of the present invention, may be either produced by a known production method or commercially available products. The method of producing maltitol includes, for example, a method in which a starch syrup containing maltose is hydrogenated by subjecting it to high pressure catalytic reduction in the presence of a catalyst, followed by purification and concentration. Examples of commercially available maltitol products include Hydrogenated Starch Hydrolysates Syrups MU-45, MU-50, MU-65, Hydrogenated Maltose Syrup MU-75, and Whole Crystalline Maltitol Ueno (Maltitol Powder) manufactured by UENO FINE CHEMICALS INDUSTRY, LTD. The method of producing sorbitol includes, for example, a method in which a glucose liquid is hydrogenated by subjecting it to high pressure catalytic reduction in the presence of a catalyst, followed by purification and concentration. Examples of commercially available sorbitol products include Sorpart, Sorbitol Ueno, and Powdered Sorbitol Ueno manufactured by UENO FINE CHEMICALS INDUSTRY, LTD.

Properties of maltitol used in the present invention may be either liquid or solid, and maltitol may be, for example, powdered maltitol or crystal maltitol produced from liquid maltitol by a known powderization or crystallization method. Properties of maltitol to be used can be appropriately selected depending on the objective dosage form.

Properties of sorbitol used in the present invention may be either liquid or solid, and sorbitol may be, for example, powdered sorbitol or crystal sorbitol produced from liquid sorbitol by a known powderization or crystallization method. Properties of sorbitol to be used can be appropriately selected depending on the objective dosage form.

With respect to the purity of maltitol used in the present invention, liquid maltitol preferably has a purity of 45% or more. Powdered maltitol preferably has a purity of 80% or more, more preferably 85% or more, and still more preferably 88% or more.

With respect to the purity of sorbitol used in the present invention, liquid sorbitol preferably has a purity of 45% or more. Powdered sorbitol preferably has a purity of 80% or more, more preferably 85% or more, and still more preferably 88% or more.

Both an AGEs production inhibitor and a 3-DG production inhibitor of the present invention contain, as active ingredients, one or more sugar alcohols selected from maltitol and sorbitol. The inhibitor may further contain excipients and the like, as long as they do not interfere with the AGEs production inhibitory effect or the 3-DG production inhibitory effect exerted by maltitol and/or sorbitol. There is no particular limitation on the proportions of maltitol and/or sorbitol in an AGEs production inhibitor or a 3-DG production inhibitor of the present invention. For example, the AGEs production inhibitor or the 3-DG production inhibitor of the present invention can contain maltitol or sorbitol in the proportion of 40% by weight or more, 45% by weight or more, 50% by weight or more, 60 o by weight or more, 70% by weight or more, 80% by weight or more, 85% by weight or more, 90% by weight or more, 95% by weight or more, or 98% by weight or more. Alternatively, the AGEs production inhibitor or 3 -DG production inhibitor of the present invention may consist only of a single ingredient which is either maltitol or sorbitol. When maltitol and sorbitol are used in combination, the total proportion of maltitol and sorbitol in the AGEs production inhibitor or 3-DG production inhibitor of the present invention can be 40% by weight or more, 45% by weight or more, 50% by weight or more, 60% by weight or more, 70% by weight or more, 80% by weight or more, 85% by weight or more, 90% by weight or more, 95% by weight or more, or 98% by weight or more. In one embodiment, one or more sugar alcohols selected from maltitol and sorbitol may be the sole active ingredient in the AGEs production inhibitor or 3-DG production inhibitor of the present invention.

The AGEs production inhibitor of the present invention enables the inhibition of the production of various AGEs including fluorescent AGEs such as pentosidine, crossline, pyrropyridine, glyoxal-derived lysine dimer (GOLD) and methylglyoxal-derived lysine dimer (MOLD); and non-fluorescent AGEs such as pyrraline, carboxymethyllysine (CML), a 3-deoxyglucosone-derived lysine dimer (DOLD) and an imidazolone compound. In one embodiment, the AGEs production inhibitor of the present invention is used to inhibit the production of pentosidine which is fluorescent AGE. In another embodiment, the AGEs production inhibitor of the present invention is used to inhibit the production of carboxymethyllysine (CML) which is non-fluorescent AGE.

The 3-DG production inhibitor of the present invention can effectively inhibit the production of, among AGEs, in particular, pyrropyridine, pyrraline, DOLD, imidazolone, 3-DG imidazolone and the like, which are mainly derived from 3-DG through inhibition of the production of 3-DG.

The medicament for the prevention/treatment of diabetes of the present invention contains, as active ingredients, one or more sugar alcohols selected from maltitol and sorbitol, as is the case with the AGEs production inhibitor and 3-DG production inhibitor of the present invention.

The proportion of maltitol or sorbitol to be contained in the medicament for the prevention/treatment of diabetes of the present invention as an active ingredient can be appropriately determined depending on the objective dosage form and the like, and may be usually from about 1 to 98% by weight, preferably from about 2 to 95% by weight, and more preferably from about 3 to 90% by weight, based on the whole amount of the medicament. When maltitol and sorbitol are used in combination, the total proportions of maltitol and sorbitol to be contained in the medicament for the prevention/treatment of diabetes of the present invention as active ingredients may be from about 1 to 98% by weight, preferably from about 2 to 95% by weight, and more preferably from about 3 to 90% by weight, based on the total amount of the medicament. In one embodiment, one or more sugar alcohols selected from maltitol and sorbitol may be the sole active ingredient in the medicament for the prevention/treatment of diabetes of the present invention.

In a preferred embodiment of the present invention, the prevention and/or treatment of diabetes or diabetic complications is/are achieved through the inhibition of the production of 3-deoxyglucosone or an advanced glycation end product.

In the present specification, "treatment" of diabetes or diabetic complications also includes the inhibition of the progression of symptoms in a patient already suffering from diabetes or diabetic complications.

A way of administration of the medicament for the prevention/treatment of diabetes of the present invention may be either of oral administration or parenteral administration. Timing of administration of the medicament is not particularly limited and may be either of before meal, during meal, after meal, or between meals. For example, the medicament is preferably administered any of before meal, during meal, after meal, or between meals, wherein the meal contains 5 grams or more of saccharides or 20 grams or more of amino acids and/or proteins, and more preferably, any of before meal, during meal, after meal, or between meals, wherein the meal contains saccharides and amino acids and/or proteins in an amount equal to or more than the above-described content. Amino acids contained in meals include lysine, arginine, etc. The medicament is preferably administered any of before meal, during meal, after meal, or between meals wherein the meal contains lysine and/or arginine, which are easily converted to AGEs, in an amount equal to or more than the above-described content. Proteins contained in meals include ovalbumin, lactalbumin, gliadin, lactic casein, soybean casein, etc. The medicament is preferably administered any of before meal, during meal, after meal, or between meals wherein the meal contains one or more of proteins selected from ovalbumin, lactalbumin, and lactic casein, which are easily converted to AGEs, in an amount equal to or more than the above-described content. In the case of oral administration, the medicament is especially preferably administered after meal or multiple times daily in small amounts, in terms of avoiding the adverse drug reaction of diarrhea due to large amounts of maltitol and/or sorbitol. Wherein, although "meal(s)" generally refer(s) to eating a food as a daily habit to take nutrients necessary for living (eating and drinking action) or to the food or drink taken, the term "meal(s)" used herein refer(s) to a set of foods or drinks taken in one eating or drinking action.

When used herein, "saccharides" refer to monosaccharides and/or disaccharides. Meanwhile, "carbohydrates" refer to carbohydrates other than dietary fiber, and include oligosaccharides, polysaccharides, sugar alcohol and the like as well as saccharides.

Dosage of the medicament for the prevention/treatment of diabetes of the present invention is appropriately selected depending on conditions such as the severity of diabetes or its complications, the severity of other diseases, age, sex and the like. The medicament may be administered in an amount that can inhibit the production of AGEs (hereinafter also referred to as an "effective amount for inhibition of AGEs production") or in an amount that can inhibit the production of 3-DG, which is a precursor of AGEs (hereinafter also referred to as an "effective amount for inhibition of 3-DG production"). The effective amount for inhibition of AGEs production and the effective amount for inhibition of 3-DG production can be appropriately determined using methods well-known to those skilled in the art (including various non-clinical and/or clinical studies).

With respect to the medicament for the prevention/treatment of diabetes of the present invention, an effective amount of maltitol and/or sorbitol for inhibition of AGEs formation or 3-DG formation may be administered in a single dose or separately administered in multiple doses at intervals. In the case of oral administration, the medicament is preferably administered separately in multiple doses in terms of laxative property. If the medicament is separately administered in multiple doses, total amount of maltitol and/or sorbitol administered per day may be the effective amount for inhibition of AGEs production or the effective amount for inhibition of 3-DG production, and the medicament is preferably administered in accordance with the number of meals. Regarding a combination of maltitol and sorbitol, "an effective amount of maltitol and sorbitol for inhibition of AGEs production or 3 -DG production" refers to an amount where the production of AGEs or 3-DG is inhibited by the administration of both ingredients. In this case, either maltitol or sorbitol alone needs not to be an effective amount for inhibition of AGEs production or 3-DG production. Quantitative ratio between maltitol and sorbitol is not particularly limited.

The medicament for the prevention/treatment of diabetes of the present invention can be administered to both individuals with diabetes or diabetic complications or healthy individuals. Especially, diabetes is highly related to AGEs and their precursor 3-DG, thus the medicament is preferably administered to individuals with diabetes. Individuals with diabetes refer to individuals with hemoglobin Alc of 6.1% or more (JDS value) and who meet any of the following criteria (1) to (3) : (1) fasting glucose levels of 126 mg/dL or more, (2) random glucose levels (regardless of fasting or after meal) of 200 mg/dL or more, and (3) 2-hour postload glucose levels of 200 mg/dL or more. Details of the diagnostic criteria of diabetes are described in the "Report of the Committee of Japan Diabetes Society on the Classification and Diagnostic Criteria of Diabetes Mellitus" (available from the website of the Society, etc.).

The medicament for the prevention/treatment of diabetes of the present invention can be used for the prevention and/or treatment of diabetic complications such as diabetic retinopathy, diabetic nephropathy, diabetic neuropathy, diabetic vascular complication, arteriosclerosis, renal failure, Alzheimer's disease, neurodegenerative disease, and cancer, which develop with diabetes. Among these diabetic complications, the medicament is preferably used for the prevention and/or treatment of diabetic retinopathy, diabetic nephropathy, and/or diabetic neuropathy with higher incidences.

Furthermore, the medicament for the prevention/treatment of diabetes of the present invention can be administered to healthy individuals to effectively prevent diabetes and its complications.

The medicament for the prevention/treatment of diabetes of the present invention can be used in various dosage forms by adding further components such as excipients, stabilizers, preservatives, buffering agents, corrigents, suspending agents, emulsifiers, flavoring agent, solubilizers, coloring agents, and viscous agents in addition to maltitol and/or sorbitol, as long as they do not interfere with the effect of the prevention and/or treatment of diabetes or diabetic complications exerted by maltitol and/or sorbitol.

Dosage forms of the medicament for the prevention/treatment of diabetes of the present invention include tablets (orally-disintegrating tablets, chewable tablets, effervescent tablets, dispersible tablets, and soluble tablets), capsules, granules (effervescent granules), powders, liquids and solutions for oral administration (elixirs, suspensions, emulsions, and lemonades), syrups (preparations for syrups), jellies for oral administration, tablets for oro-mucosal application (troches/lozenges, sublingual tablets, buccal tablets, mucoadhesive tablets, and medicated chewing gums), sprays for oro-mucosal application, semi-solid preparations for oro-mucosal application, preparations for gargles, injections (parenteral infusions, implants/injections, and prolonged release injections), dialysis agents (peritoneal dialysis agents and hemodialysis agents), inhalations (dry powder inhalers, inhalation solutions, and metered-dose inhalers), suppositories for rectal application, semi-solid preparations for rectal application, enemas for rectal application, ophthalmic preparations, ophthalmic ointments, ear preparations, nasal preparations (nasal dry powder inhalers and nasal solutions), tablets for vaginal use, suppositories for vaginal use, solid dosage forms for cutaneous application (powders for cutaneous application), liquids and solutions for cutaneous application (liniments and lotions), sprays for cutaneous application (aerosols for cutaneous application and pump sprays for cutaneous application), ointments, creams, gels, patches (tapes/plasters and cataplasms/gel patches), etc. Among these dosage forms, in terms of being easy to administer orally, tablets, capsules, granules, powders, liquids and solutions for oral administration, syrups, jellies for oral administration, tablets for oro-mucosal application, sprays for oro-mucosal application, semi-solid preparations for oro-mucosal application, and preparations for gargles are preferable, and in terms of being easy to be absorbed in vivo, tablets, capsules, granules, and powders are more preferable.

The medicament for the prevention/treatment of diabetes of the present invention can be used in dosage forms in which AGEs production inhibitor or 3-DG production inhibitor of the present invention is added to preparations related to crude drugs such as extracts, pills, spirits, infusions and decoctions, teabags, tinctures, aromatic waters, and fluidextracts. These dosage forms are appropriately selected depending on conditions such as the severity of diabetes or its complications, the severity of other diseases, age, sex and the like.

Furthermore, the medicament for the prevention/treatment of diabetes of the present invention can further contain other drugs having AGEs production inhibitory effects. These drugs include, for example, insulin sensitizers, antidyslipidemic agents, angiotensin II1 receptor blockers, angiotensin-converting enzyme inhibitors, metformin and the like.

The medicament for the prevention/treatment of diabetes of the present invention can further contain natural products or naturally-derived substances having AGEs production inhibitory effects. These natural products or naturally-derived substances include, for example, herbs such as Saururaceae, Crataegus oxyacantha, chamomile, and grape leaves, cherry blossoms, the style and stigma of corns, seeds of Plantago major, flowers of horse chestnut, peony and roses, fruits of Japanese apricot, Vitis coignetiae, purple chrysanthemum, wheat germ, and wheat germ-derived polyamine, mangosteen and the like.

AGEs production inhibitor or 3-DG production inhibitor of the present invention, or one or more sugar alcohols selected from maltitol and sorbitol which are active ingredients of the inhibitors can also be used to prevent and/or treat diabetes or diabetic complications in non-human animals. Therefore, in one embodiment of the present invention, a medicament for non-human animals containing one or more sugar alcohols selected from maltitol and sorbitol for the prevention and/or treatment of diabetes or diabetic complications in non-human animals (hereinafter also referred to as a "medicament for non-human animals of the present invention") is provided. In addition, a method of preventing and/or treating diabetes or diabetic complications in non-human animals, which comprises administering the medicament for non-human animals or AGEs production inhibitor or 3-DG production inhibitor of the present invention to non-human animals, is also provided. Examples of non-human animals include livestock such as cattle, horses, pigs, sheep, goats and chickens; and animals that are reared as pets such as dogs and cats.

Amounts of maltitol and/or sorbitol contained in the medicament for non-human animals of the present invention, a way of administration, timing of administration, dosage, and dosage forms of the medicament, other drugs, natural products and naturally-derived substances that can be used in combination with maltitol and/or sorbitol in the medicaments; and a way of administration, timing of administration, dosage and the like when AGEs production inhibitor or 3-DG production inhibitor of the present invention is administered to non-human animals are same as those described above regarding "the medicament for the prevention/treatment of diabetes of the present invention".

Furthermore, AGEs production inhibitor or 3-DG production inhibitor of the present invention can also be used as food additives. Therefore, by mixing or adding AGEs production inhibitor or 3-DG production inhibitor of the present invention to food or drink, functional food or drink that inhibits the production of AGEs or 3-DG can be provided. By using AGEs production inhibitor or 3-DG production inhibitor of the present invention as components, supplements that inhibit the production of AGEs or 3-DG can also be manufactured. In this case, excipients and the like can be appropriately compounded depending on the dosage forms of supplements to be manufactured.

Examples of the subject to which the method of preventing/treating diabetes of the present invention is applied include humans and non-human animals, for example, livestock such as cattle, horses, pigs, sheep, goats and chickens; and animals that are reared as pets such as dogs and cats.

In one embodiment of the present invention, one or more sugar alcohols selected from maltitol and sorbitol are provided as a solution containing the sugar alcohol, or as a food or drink to which the sugar alcohol has been added. In one embodiment, a medicament for the prevention/treatment of diabetes, a medicament for non-human animals, an AGEs production inhibitor and a 3-DG production inhibitor of the present invention can be in the form of a solution containing one or more sugar alcohols selected from maltitol and sorbitol, or a form of food or drink to which the sugar alcohol has been added. Examples of such form of the solution include liquids and solutions for oral administration, preparations for gargles, injections, enemas for rectal application, ophthalmic preparations, nasal solutions, liquids and solutions for cutaneous application, and the like.

### EXAMPLES

The present invention will be further described below by way of Examples.

### Test Example 1

### (a) Measurements of 3-DG

### (1) Preparation of samples

Amino acids, carbohydrates and a phosphate buffer solution were charged in a 30 mL glass test tube in each amount shown in Table 1, followed by sealing and stirring. After reacting in an autoclave at 120°C for 60 minutes, the reaction solution was cooled and then filtered through a 0.2 µm filter. The filtrate thus obtained was used as a sample. The amino acids and carbohydrates used are as follows.

### [Amino Acids]

Lysine (N-α-(t-butoxycarbonyl)-L-lysine, manufactured by WATANABE CHEMICAL INDUSTRIES, LTD.)
Arginine (N-α-(t-butoxycarbonyl)-L-arginine, manufactured by WATANABE CHEMICAL INDUSTRIES, LTD.)

### [Carbohydrates]

Sucrose (manufactured by Taito Co., Ltd.)
Maltitol (Maltitol Powder Ueno 60M (having a maltitol purity of 90%), manufactured by UENO FINE CHEMICALS INDUSTRY, LTD.)

**Table 1**

| | Lysine (g) | Arginine (g) | Sucrose (g) | Maltitol (g) | Phosphate buffer solution (mL) |
|---|---|---|---|---|---|
| Test section 1 | 0.2 | 0.2 | 3.40 | 0 | 20 |
| Test section 2 | 0.2 | 0.2 | 0 | 3.40 | 20 |
| Test section 3 | 0.2 | 0.2 | 3.40 | 3.40 | 20 |
| Test section 4 | 0.2 | 0.2 | 0 | 0 | 20 |

| | | | | | |
|---|---|---|---|---|---|
| *The amount of maltitol in the table was expressed as the weight (g) of "Maltitol Powder Ueno 60M". | | | | | |

### (2) Test Methods

To 2 mL of a sample, the same amount of a 60% perchloric acid solution was added to deproteinize the sample. The sample was neutralized with 4 mL of an aqueous saturated sodium hydrogen carbonate solution and 0.8 mL of a 0.1% methanol solution of 2,3-diaminonaphthalene was added, and then the mixture was reacted at 4°C for 16 hours. Next, the reactant was extracted with 4.4 mL of ethyl acetate, concentrated to dryness using an evaporator, dissolved in 5 mL of methanol, and then filtered through a 0.45 µm filter. The filtrate was subjected to high-performance liquid chromatography and 3-DG was quantitatively determined under the following measurement conditions.

### [Measurement Conditions of High-Performance Liquid Chromatography]

UV: 268 nm
Injection amount: 20 µL
Column: TSK-GEL, ODS-80Ts (4.6 x 150 mm, manufactured by TOSOH

### CORPORATION)

Column temperature: 30°C
Mobile phase: 50 mM phosphoric acid:methanol:acetonitrile = 7:1.5:1.5
Flow rate: 1 mL/min.

### (3) Results

The results of quantitative determination of 3-DG are shown in Table 2.

**Table 2**

| | 3-DG (nmol/1 g of amino acid) |
|---|---|
| Test section 1 (Amino acid + Sucrose) | 151 |
| Test section 2 (Amino acid + Maltitol) | 36 |
| Test section 3 (Amino acid + Sucrose + Maltitol) | 121 |
| Test section 4 (No carbohydrate) | 0 |

### (b) Measurements of fluorescent AGEs

### (1) Preparation of samples

The same samples as those used in the above measurement of 3-DG were used.

### (2) Test Methods

After weighing 2.5 mg of a standard substance perylene, the standard substance perylene was fully dissolved in 50 mL of deaerated ethanol added thereto to prepare a perylene solution. The perylene solution diluted to the concentration of 10 µM was exposed to excitation light with a wavelength of 386 nm and then the area was calculated by measuring a fluorescence intensity ranging from 410 to 600 nm. Meanwhile, the sample was exposed to excitation light with a wavelength of 380 nm and then the area was calculated by measuring a fluorescence intensity ranging from 400 to 600 nm. By considering the area measurement value of the sample as a value relative to the area value of the 10 µM perylene solution, fluorescent AGEs were quantitatively determined.

### (3) Results

The results of quantitative determination of fluorescent AGEs are shown in Table 3.

**Table 3**

| | Fluorescent AGEs (nmol/1 g of amino acid) |
|---|---|
| Test section 1 (Amino acid + Sucrose) | 79 |
| Test section 2 (Amino acid + Maltitol) | 39 |
| Test section 3 (Amino acid + Sucrose + Maltitol) | 92 |
| Test section 4 (No carbohydrate) | 0 |

### (c) Measurements of Pentosidine

### (1) Preparation of samples

The same samples as those used in the above measurement of 3-DG were used.

### (2) Test Methods

Using a pentosidine measurement kit "FSK Pentosidine (registered trademark)" (manufactured by Fushimi Pharmaceutical Co., Ltd.), quantitative determination of pentosidine was performed. Thirty (30) or more minutes before use, reagents and pentosidine solid-phase plates were put to room temperature. After dilution of the sample, a 50 µL aliquot of the sample was dispensed to a pentosidine solid plate attached to the kit, and 50 µL of a first antibody solution was dispensed into wells other than the wells for measurement of reagent blank value. After the sample and the antibody solution were mixed by shaking the plate at 300 rpm for 1 minute, the solution was reacted at 37°C for 1 hour. Next, the reaction solution was discarded, and 200 µL of rinsing fluid was dispensed to wash the plate. The washing was performed for 3 times. After liquid on the plate was removed, 100 µL of a second antibody solution was dispensed into wells other than the wells for measurement of reagent blank value. After the solution was mixed by shaking the plate at 300 rpm for 1 minute, the solution was reacted at 25°C for 1 hour. Then, the reaction solution was discarded, and the plate was washed for 3 times with 200 µL of rinsing fluid again. Liquid on the plate was removed, and 100 µL of a color coupler was dispensed and left to stand for 10 minutes under a light shielding condition. Finally, 100 µL of a reaction stop solution was dispensed, and within 10 minutes after the dispensing, measurement was performed at main wavelength 450 nm/reference wavelength 630 nm using a micro-plate reader. The concentration of pentosidine was determined using the calibration curve of a pentosidine standard solution, based on measurement values of the sample.

### (3) Results

The results of quantitative determination of pentosidine are shown in Table 4.

**Table 4**

| | Pentosidine (nmol/1 g of amino acid) |
|---|---|
| Test section 1 (Amino acid + Sucrose) | 8.4 |
| Test section 2 (Amino acid + Maltitol) | 3.4 |
| Test section 3 (Amino acid + Sucrose + Maltitol) | 13.3 |
| Test section 4 (No carbohydrate) | 0.3 |

### (d) Measurement of CML

### (1) Preparation of samples

The same samples as those used in the above measurement of 3-DG were used.

### (2) Test Methods

Using CircuLex CML/Nε-(carboxymethyl)lysine ELISA kit (manufactured by CycLex Co., Ltd.), quantitative determination of CML was performed. Thirty (30) or more minutes before use, reagents and CML solid-phase wells were put to room temperature. By adding 1 mL of ultrapure water (Milli-Q water) to a CML-HSA standard powder, a master standard was prepared. By performing dilution with standard dilution buffers, standards 1 to 7 were prepared. The sample was diluted as necessary with sample dilution buffers. A CML-HSA standard and 60 µL of the sample were dispensed into a 96-well plate, which is not an accessory to the kit. Next, ultrapure water (Milli-Q water) was added to a first antibody powder, and by diluting with sample dilution buffers, a first antibody solution was prepared, and then 60 µL of the antibody solution was dispensed into the plate and mixed. After 1.00 µL of the mixture was added to CML solid-phase wells, the mixture was reacted at 25°C for 1 hour while being shaken at 300 rpm. Then, the reaction solution was discarded, and 350 µL of rinsing fluid was dispensed to wash the plate. The washing was performed for 4 times. After liquid on the plate was removed, 100 µL of a second antibody solution was dispensed and reacted at 25°C for 1 hour while being shaken at 300 rpm. The reaction solution was discarded, and the plate was washed for 4 times with 350 µL of rinsing fluid again. Liquid on the plate was removed, and 100 µL of a color coupler was dispensed. Then, the solution was left to stand for 10 minutes under a light shielding condition while being shaken at 300 rpm. Finally, 100 µL of a reaction stop solution was dispensed, and within 30 minutes after the dispensing, measurement was performed at main wavelength 450 nm/reference wavelength 595 nm using a micro-plate reader. The concentration of CML was determined using the calibration curve of a CML standard solution, based on measurement values of the sample.

### (3) Results

The results of quantitative determination of CML are shown in Table 5.

**Table 5**

| | CML (nmol/1 g of amino acid) |
|---|---|
| Test section 1 (Amino acid + Sucrose) | 17.4 |
| Test section 2 (Amino acid + Maltitol) | 15.0 |
| Test section 3 (Amino acid + Sucrose + Maltitol) | 18.3 |
| Test section 4 (No carbohydrate) | 17.5 |

### Evaluation of AGEs Production Inhibitory Effect

Compared with the test section 1 containing only sucrose as a carbohydrate, in the test section 2 containing only maltitol as a carbohydrate, the production of 3-DG was significantly decreased and each production of fluorescent AGEs, pentosidine and CML was also decreased. In the test section 3 containing sucrose and maltitol as carbohydrates, the productions of 3-DG and various AGEs were decreased compared with the test section 1. These results reveal 3-DG and AGEs production inhibitory effects of maltitol in the presence of amino acids.

### Test Example 2

### (1) Preparation of samples

Food-derived proteins, carbohydrates and a phosphate buffer solution were charged in a 30 mL glass test tube in each amount shown in Table 6, followed by sealing and stirring, and then the mixture was reacted in an autoclave at 120°C for 60 minutes. Next, the reactant was fractionated by centrifugation at 3, 000 rpm for 3 hours using an ultrafiltration unit (VIVA SPIN 20, manufactured by Sartorius Corporation). The obtained low molecular weight fraction was used as a sample for determination of 3-DG, while the obtained high molecular weight fraction was used as a sample for the determination of fluorescent AGEs, RAGE agonist activity and RAGE antagonist activity. Food-derived proteins and carbohydrates used are as follows:

### [Food-Derived Proteins]

Ovalbumin (manufactured by Tokyo Chemical Industry Co. , Ltd.)
Lactalbumin (manufactured by Tokyo Chemical Industry Co., Ltd.)
Gliadin (manufactured by Tokyo Chemical Industry Co., Ltd.) Lactic casein (manufactured by KATAYAMA CHEMICAL, INC.) Soybean casein (manufactured by KISHIDA CHEMICAL Co., Ltd.)

### [Carbohydrates]

Sucrose (manufactured by Taito Co., Ltd.)
Maltitol (Maltitol Powder Ueno 60M (having a maltitol purity of 90%), manufactured by UENO FINE CHEMICALS INDUSTRY, LTD.)

**Table 6**

| | Ovalbumin (g) | Lactalbumin (g) | Gliadin (g) | Lactic casein (g) | Soybean casein (g) | Sucrose (g) | Maltitol (g) | Phosphate buffer solution (mL) |
|---|---|---|---|---|---|---|---|---|
| Test section 1 | 0.05 | 0 | 0 | 0 | 0 | 3.4 | 0 | 20 |
| Test section 2 | 0.05 | 0 | 0 | 0 | 0 | 0 | 3.4 | 20 |
| Test section 3 | 0.05 | 0 | 0 | 0 | 0 | 0 | 0 | 20 |
| Test section 4 | 0 | 0.05 | 0 | 0 | 0 | 3.4 | 0 | 20 |
| Test section 5 | 0 | 0.05 | 0 | 0 | 0 | 0 | 3.4 | 20 |
| Test section 6 | 0 | 0.05 | 0 | 0 | 0 | 0 | 0 | 20 |
| Test section 7 | 0 | 0 | 0.1 | 0 | 0 | 3.4 | 0 | 20 |
| Test section 8 | 0 | 0 | 0.1 | 0 | 0 | 0 | 3.4 | 20 |
| Test section 9 | 0 | 0 | 0.1 | 0 | 0 | 0 | 0 | 20 |
| Test section 10 | 0 | 0 | 0 | 0.01 | 0 | 3.4 | 0 | 20 |
| Test section 11 | 0 | 0 | 0 | 0.01 | 0 | 0 | 3.4 | 20 |
| Test section 12 | 0 | 0 | 0 | 0.01 | 0 | 0 | 0 | 20 |
| Test section 13 | 0 | 0 | 0 | 0 | 0.01 | 3.4 | 0 | 20 |
| Test section 14 | 0 | 0 | 0 | 0 | 0.01 | 0 | 3.4 | 20 |
| Test section 15 | 0 | 0 | 0 | 0 | 0.01 | 0 | 0 | 20 |

| | | | | | | | | |
|---|---|---|---|---|---|---|---|---|
| *The amount of maltitol in the table was expressed as the weight (g) of "Powdered Maltitol Ueno 60M". | | | | | | | | |

### (2) Test Methods

### (a) Quantitative Determination of 3-DG

In the same manner as in Test Example 1, except that the low molecular weight fraction prepared as mentioned above was used as a sample, quantitative determination of 3-DG was performed.

### (b) Quantitative Determination of Fluorescent AGEs

After 0.4 g of a high molecular weight fraction and 8 mL of 6N hydrochloric acid were charged in an ampule and the air in the ampule was replaced with nitrogen, hydrolysis was performed at 110°C for 16 hours in a heat block. After reaction, 8 mL of 5N sodium hydroxide was added, and pH was adjusted using 1N hydrochloric acid and 1N sodium hydroxide to make pH within a range of 7.2 to 7.6. Then, the solution was filtered through a 0.45 µm filter. Using the thus obtained filtrate as a sample, fluorescent AGEs were quantitatively determined using the following methods. After weighing 2.5 mg of a standard substance perylene, the standard substance perylene was fully dissolved in 10 mL of deaerated ethanol added thereto to prepare a perylene solution. The perylene solution diluted to the concentration of 10 µM was exposed to excitation light with a wavelength of 386 nm and then the area was calculated by measuring a fluorescence intensity ranging from 410 to 600 nm. Meanwhile, the sample was exposed to excitation light with a wavelength of 380 nm and then the area was calculated by measuring a fluorescence intensity ranging from 400 to 600 nm. By considering the area measurement value of the sample as a value relative to the area value of the 10 µM perylene solution, fluorescent AGEs were determined.

### (c) Evaluation of RAGE Agonist Activity

Using Luciferase Assay System (manufactured by Promega Corporation), evaluation of RAGE agonist activity was performed. (Day 1) According to the methods disclosed in Diabetes, 2006, vol. 55, pp.2510-2522, C6 glioma cells into which pNF-κB-Luc plasmid (manufactured by Stratagene Corporation) and human RAGE plasmid (including full-length human RAGE cDNA)have been introduced were seeded at 100 µL/well into a 96-well plate and cultured in a CO₂ incubator until the cells were confluent. For the culture, 10% FBS/DMEM medium was used. (Day 2) The medium was removed, and after wells were washed with 300 µL of serum-free DMEM medium, 100 µL of 0.1% FBS/DMEM medium was slowly added. Then, the medium was incubated overnight in a CO₂ incubator. (Day 3) The sample was diluted using the medium, and 50 µL of the diluted sample was added to each well. AGE-BSA in which AGE was formed with glyceraldehydes (50 µg/mL) was added to a control well. After incubation in a CO₂ incubator for 4 hours, the medium was removed and wells were washed 2 times with 300 µL of PBS (-). Then, 25 µL of 1 x lysis buffer was added on ice. After the plate was shaken for 15 seconds, 20 µL of lysate was charged in a new white plate. Further 100 µL of a luciferase assay reagent was added, and a fluorescence intensity was measured by a luminometer. Using 5 µL of lysate, quantitative determination of protein was also performed.

### (d) Evaluation of RAGE Antagonist Activity

Using Luciferase Assay System (manufactured by Promega Corporation), evaluation of RAGE antagonist activity was performed. (Day 1) According to the methods disclosed in Diabetes, 2006, vol.55, pp.2510-2522, C6 glioma cells into which pNF-κB-Luc plasmid (manufactured by Stratagene Corporation) and human RAGE plasmid (including full-length human RAGE cDNA) have been introduced were seeded at 100 µL/well into a 96-well plate and cultured in a CO₂ incubator until the cells were confluent. For the culture, 10% FBS/DMEM medium was used. (Day 2) The medium was removed, and after wells were washed with 300 µL of serum-free DMEM medium, 100 µL of 0.1% FBS/DMEM medium was slowly added. Then, the medium was incubated overnight in a CO₂ incubator. (Day 3) The sample was diluted using the medium, and 50 µL of the diluted sample, AGE-BSA in which AGE was formed with glyceraldehydes (50 µg/mL) as a control, and the mixture of diluted sample and AGE-BSA were added to wells. After incubation in a CO₂ incubator for 4 hours, the medium was removed and wells were washed 2 times with 300 µL of PBS (-). Then, 25 µL of 1 x lysis buffer was added on ice. After the plate was shaken for 15 seconds, 20 µL of lysate was charged in a new white plate. Further 100 µL of a luciferase assay reagent was added, and a fluorescence intensity was measured by a luminometer. Using 5 µL of lysate, quantitative determination of protein was also performed.

### (3) Results

In the combination with each of food-derived proteins, i.e. ovalbumin, lactalbumin, gliadin, lactic casein and soybean casein, in the test sections 2, 5, 8, 11 and 14 containing maltitol as a carbohydrate, the production of 3-DG was significantly decreased and the production of AGEs was also decreased, compared with in the test sections 1, 4, 7, 10 and 13 containing sucrose as a carbohydrate. In the test sections 2, 5 and 11 containing maltitol as a carbohydrate, RAGE agonist activity was decreased, compared with the test sections 1, 4 and 10 containing sucrose as a carbohydrate. In the test sections 2 and 5 containing maltitol as a carbohydrate, RAGE antagonist activity was decreased, compared with the test sections 1 and 4 containing sucrose as a carbohydrate. These results reveal AGEs and 3-DG production inhibitory effects of maltitol in the presence of food-derived proteins. The results of quantitative determination of AGEs and 3-DG are shown in Table 7.

**Table 7**

| | Fluorescent AGEs (nmol/1 g of protein) | 3-DG (nmol/1 g of protein) | RAGE agonist activity* | RAGE antagonist activity* |
|---|---|---|---|---|
| Test section 1 (Ovalbumin + Sucrose) | 168 | 4,532 | 1.66 | 1.80 |
| Test section 2 (Ovalbumin + Maltitol) | 136 | 523 | 1.42 | 1.74 |
| Test section 3 (Ovalbumin) | 43 | 0 | 1.22 | 1.61 |
| Test section 4 (Lactalbumin + Sucrose) | 119 | 4,758 | 0.81 | 0.58 |
| Test section 5 (Lactalbumin + Maltitol) | 105 | 656 | 0.79 | 0.54 |
| Test section 6 (Lactalbumin) | 55 | 373 | 0.57 | 0.81 |
| Test section 7 (Gliadin + Sucrose) | 649 | 2,645 | 0.53 | 1.00 |
| Test section 8 (Gliadin + Maltitol) | 632 | 240 | 0.51 | 0.90 |
| Test section 9 (Gliadin) | 500 | 65 | 0.52 | 1.00 |
| Test section 10 (Lactic casein + Sucrose) | 100 | 22,464 | 0.53 | 1.50 |
| Test section 11 (Lactic casein + Maltitol) | 84 | 2,734 | 0.51 | 1.42 |
| Test section 12 (Lactic casein) | 38 | 0 | 0.54 | 1.02 |
| Test section 13 (Soybean casein + Sucrose) | 18 | 24,285 | 0.34 | 1.01 |
| Test section 14 (Soybean casein + Maltitol) | 20 | 1,446 | 0.24 | 0.99 |
| Test section 15 (Soybean casein) | 13 | 199 | 0.31 | 1.02 |

| | | | | |
|---|---|---|---|---|
| *The values of the respective samples (for RAGE agonist activity), and the liquid mixture of the respective samples and AGE-BSA (for RAGE antagonist activity) relative to the value of control (AGE-BSA) were shown, assuming that the control value is 1. The value when the amount of protein is 10 µg/mL for ovalbumin and lactalbumin, the value when the amount of protein is 0.1 µg/mL for gliadin and soybean casein, and the value when the amount of protein is 1 µg/mL for lactic casein were shown, respectively. | | | | |

### Test Example 3

### (1) Preparation of Samples

Amino acid or biological protein, carbohydrates and a phosphate buffer solution were charged in a 50 mL polypropylene centrifuge tube in each amount shown in Table 8, followed by sealing and stirring, and then the mixture was reacted in an incubator at 37°C for 42 days. Next, the reactant was fractionated by centrifugation at 3,000 rpm for 3 hours using an ultrafiltration unit (VIVA SPIN 20, manufactured by Sartorius Corporation). The obtained low molecular weight fraction was used as a sample for determination of 3-DG, while the obtained high molecular weight fraction was used as a sample for the determination of fluorescent AGEs. Amino acid, biological protein and carbohydrates used are as follows:

### [Amino Acids]

Mixture of lysine (N-α-(t-butoxycarbonyl)-L-lysine, manufactured by WATANABE CHEMICAL INDUSTRIES, LTD.) and arginine (N-α-(t-butoxycarbonyl)-L-arginine, manufactured by WATANABE CHEMICAL INDUSTRIES, LTD.) in a ratio of 1:1

### [Biological proteins]

Bovine serum albumin (manufactured by Sigma-Aldrich)

### [Carbohydrates]

Glucose (Special grade reagent, manufactured by Wako Pure Chemical Industries, Ltd.)
Maltitol (Maltitol Powder Ueno 60M (maltitol purity 90%), manufactured by UENO FINE CHEMICALS INDUSTRY, LTD.)

**Table 8**

| | Amino acid (g) | Bovine serum albumin (g) | Glucose (g) | Maltitol (g) | Phosphate buffer solution (mL) |
|---|---|---|---|---|---|
| Test section 1 | 0.4 | 0 | 1.8 | 0 | 20 |
| Test section 2 | 0.4 | 0 | 0 | 3.4 | 20 |
| Test section 3 | 0.4 | 0 | 0 | 0 | 20 |
| Test section 4 | 0 | 0.4 | 1.8 | 0 | 20 |
| Test section 5 | 0 | 0.4 | 0 | 3.4 | 20 |
| Test section 6 | 0 | 0.4 | 0 | 0 | 20 |

| | | | | | |
|---|---|---|---|---|---|
| *The amount of maltitol in the table was expressed as the weight (g) of "Maltitol Powder Ueno 60M". | | | | | |

### (2) Test Methods

In the same manner as in Test Example 1, except that the low molecular weight fraction prepared as mentioned above was used as a sample, quantitative determination of 3-DG was performed. In the same manner as in Test Example 2, except that the high molecular weight fraction prepared as mentioned above was used as a sample, determination of fluorescent AGEs was performed.

### (3) Results

In the combination with each of amino acids and bovine serum albumin, in the test sections 2 and 5 containing maltitol as a carbohydrate, the production of 3-DG was significantly decreased and the production of AGEs was also decreased, compared with in the test sections 1 and 4 containing glucose as a carbohydrate. These results reveal AGEs and 3-DG production inhibitory effects of maltitol in the presence of amino acid or food-derived protein. The results of quantitative determination of AGEs and 3-DG are shown in Table 9.

**Table 9**

| | Fluorescent AGEs (nmol/1 g of protein) | 3-DG (nmol/1 g of protein) |
|---|---|---|
| Test section 1 (Amino acid + Glucose) | 91 | 22,761 |
| Test section 2 (Amino acid + Maltitol) | 2 | 127 |
| Test section 3 (Amino acid) | 0.0 | 0.0 |
| Test section 4 (Bovine serum albumin + Glucose) | 46 | 29,938 |
| Test section 5 (Bovine serum albumin + Maltitol) | 7 | 1,181 |
| Test section 6 (Bovine serum albumin) | 13 | 9 |

### Test Example 4

### (1) Preparation of Samples

Amino acid, carbohydrates and a phosphate buffer solution were charged in a 50 mL polypropylene centrifuge tube in each amount shown in Table 10, followed by sealing and stirring, and then the mixture was reacted in an incubator at 37°C for 42 days. Next, the reactant was filtered through a 0.2 µm filter and the obtained filtrate was used as a sample. Amino acid and carbohydrates used are as follows:

### [Amino Acids]

Mixture of lysine (N-α-(t-butoxycarbonyl)-L-lysine, manufactured by WATANABE CHEMICAL INDUSTRIES, LTD.) and arginine (N-α-(t-butoxycarbonyl)-L-arginine, manufactured by WATANABE CHEMICAL INDUSTRIES, LTD.) in a ratio of 1:1

### [Carbohydrates]

Glucose (Special grade reagent, manufactured by Wako Pure Chemical Industries, Ltd.)
Fructose (KC special grade, manufactured by KATAYAMA CHEMICAL, INC.)
Sorbitol (Sigma-Aldrich Japan, special grade, manufactured by Sigma-Aldrich Japan, LLC)

**Table 10**

| | Amino acid (g) | Glucose (g) | Fructose (g) | Sorbitol (g) | Phosphate buffer solution (mL) |
|---|---|---|---|---|---|
| Test section 1 | 0.4 | 1.8 | 0 | 0 | 20 |
| Test section 2 | 0.4 | 0 | 1.8 | 0 | 20 |
| Test section 3 | 0.4 | 0 | 0 | 1.8 | 20 |

### (2) Test Methods

With respect to the samples prepared as mentioned above, 3-DG, fluorescent AGEs, pentosidine, CML, RAGE agonist activity and RAGE antagonist activity were evaluated by the following methods.

### (a) Quantitative Determination of 3-DG

To 2 mL of a sample, the same amount of a 60% perchloric acid solution was added to deproteinize the sample. The sample was neutralized with 4 mL of an aqueous saturated sodium hydrogen carbonate solution and 0.8 mL of a 0.1% methanol solution of 2,3-diaminonaphthalene was added, and then the mixture was reacted at 4°C for 16 hours. Next, the reactant was extracted with 4.4 mL of ethyl acetate, concentrated to dryness using an evaporator, dissolved in 5 mL of methanol, and then filtered through a 0.45 µm filter. The filtrate was subjected to high-performance liquid chromatography and 3-DG was quantitatively determined under the following measurement conditions.

### [Measurement Conditions of High-Performance Liquid Chromatography]

UV: 268 nm
Injection amount: 20 µL
Column: TSK-GEL, ODS-80Ts (4.6 x 150 mm, manufactured by TOSOH

### CORPORATION)

Column temperature: 30°C
Mobile phase: 50 mM phosphoric acid:methanol:acetonitrile = 7:1.5:1.5
Flow rate: 1 mL/min.

### (b) Quantitative Determination of Fluorescent AGEs

After weighing 2.5 mg of a standard substance perylene, the standard substance perylene was fully dissolved in 50 mL of deaerated ethanol added thereto to prepare a perylene solution. The perylene solution diluted to the concentration of 10 µM was exposed to excitation light with a wavelength of 386 nm and then the area was calculated by measuring a fluorescence intensity ranging from 410 to 600 nm. Meanwhile, the sample was exposed to excitation light with a wavelength of 380 nm and then the area was calculated by measuring a fluorescence intensity ranging from 400 to 600 nm. By considering the area measurement value of the sample as a value relative to the area value of the 10 µM perylene solution, fluorescent AGEs were quantitatively determined.

### (c) Quantitative Determination of Pentosidine

Using a pentosidine measurement kit "FSK Pentosidine (registered trademark)" (manufactured by Fushimi Pharmaceutical Co., Ltd.), quantitative determination of pentosidine was performed in the following manner. Thirty (30) or more minutes before use, reagents and pentosidine solid-phase plates were put to room temperature. After dilution of the sample, a 50 µL aliquot of the sample was dispensed to a pentosidine solid-phase plate attached to the kit, and 50 µL of a first antibody solution was dispensed into wells other than the wells for measurement of reagent blank value. After the sample and the antibody solution were mixed by shaking the plate at 300 rpm for 1 minute, the solution was reacted at 37°C for 1 hour. Next, the reaction solution was discarded, and 200 µL of rinsing fluid was dispensed to wash the plate. The washing was performed for 3 times. After liquid on the plate was removed, 100 µL of a second antibody solution was dispensed into wells other than the wells for measurement of reagent blank value. After the solution was mixed by shaking the plate at 300 rpm for 1 minute, the solution was reacted at 25°C for 1 hour. Then, the reaction solution was discarded, and the plate was washed for 3 times with 200 µL of rinsing fluid again. Liquid on the plate was removed, and 100 µL of a color coupler was dispensed and left to stand for 10 minutes under a light shielding condition. Finally, 100 µL of a reaction stop solution was dispensed, and within 10 minutes after the dispensing, measurement was performed at main wavelength 450 nm/reference wavelength 630 nm using a micro-plate reader. The concentration of pentosidine was determined using the calibration curve of a pentosidine standard solution, based on measurement values of the sample.

### (d) Quantitative Determination of CML

Using CircuLex CML/Nε-(carboxymethyl)lysine ELISA kit (manufactured by CycLex Co. , Ltd.), quantitative determination of CML was performed in the following manner. Thirty (30) or more minutes before use, reagents and CML solid-phase wells were put to room temperature. By adding 1 mL of ultrapure water (Milli-Q water) to a CML-HSA standard powder, a master standard was prepared. By performing dilution with standard dilution buffers, standards 1 to 7 were prepared. The sample was diluted as necessary with sample dilution buffers. A CML-HSA standard and 60 µL of the sample were dispensed into a 96-well plate, which is not an accessory to the kit. Next, ultrapure water (Milli-Q water) was added to a first antibody powder, and by diluting with sample dilution buffers, a first antibody solution was prepared; 60 µL of the antibody solution was dispensed into the plate and mixed. After 100 µL of the mixture was added to CML solid-phase wells, the mixture was reacted at 25°C for 1 hour while being shaken at 300 rpm. Then, the reaction solution was discarded, and 350 µL of rinsing fluid was dispensed to wash the plate. The washing was performed for 4 times. After liquid on the plate was removed, 100 µL of a second antibody solution was dispensed and reacted at 25°C for 1 hour while being shaken at 300 rpm. The reaction solution was discarded, and the plate was washed for 4 times with 350 µL of rinsing fluid again. Liquid on the plate was removed, and 100 µL of a color coupler was dispensed. Then, the solution was left to stand for 10 minutes under a light shielding condition while being shaken at 300 rpm. Finally, 100 µL of a reaction stop solution was dispensed, and within 30 minutes after the dispensing, measurement was performed at main wavelength 450 nm/reference wavelength 595 nm using a micro-plate reader. The concentration of CML was determined using the calibration curve of a CML standard solution, based on measurement values of the sample.

### (e) Evaluation of RAGE Agonist Activity

Using Luciferase Assay System (manufactured by Promega Corporation) , evaluation of RAGE agonist activity was performed. (Day 1) According to the methods disclosed in Diabetes, 2006, vol. 55, pp.2510-2522, C6 glioma cells into which pNF-κB-Luc plasmid (manufactured by Stratagene Corporation) and human RAGE plasmid (including full-length human RAGE cDNA) have been introduced were seeded at 100 µL/well into a 96-well plate and cultured in a CO₂ incubator until the cells were confluent. For the culture, 10% FBS/DMEM medium was used. (Day 2) The medium was removed, and after wells were washed with 300 µL of serum-free DMEM medium, 100 µL of 0.1% FBS/DMEM medium was slowly added. Then, the medium was incubated overnight in a CO₂ incubator. (Day 3) The sample was diluted using the medium, and 50 µL of the diluted sample was added to each well. AGE-BSA in which AGE was formed with glyceraldehydes (50 µg/mL) was added to a control well. After incubation in a CO₂ incubator for 4 hours, the medium was removed and wells were washed 2 times with 300 µL of PBS (-) . Then, 25 µL of 1 x lysis buffer was added on ice. After the plate was shaken for 15 seconds, 20 µL of lysate was charged in a new white plate. Further 100 µL of a luciferase assay reagent was added, and a fluorescence intensity was measured by a luminometer. Using 5 µL of lysate, quantitative determination of protein was also performed.

### (f) Evaluation of RAGE Antagonist Activity

Using Luciferase Assay System (manufactured by Promega Corporation), evaluation of RAGE antagonist activity was performed. (Day 1) According to the methods disclosed in Diabetes, 2006, vol.55, pp.2510-2522, C6 glioma cells into which pNF-κB-Luc plasmid (manufactured by Stratagene Corporation) and human RAGE plasmid (including full-length human RAGE cDNA) have been introduced were seeded at 100 µL/well into a 96-well plate and cultured in a CO₂ incubator until the cells were confluent. For the culture, 10% FBS/DMEM medium was used. (Day 2) The medium was removed, and after wells were washed with 300 µL of serum-free DMEM medium, 100 µL of 0.1% FBS/DMEM medium was slowly added. Then, the medium was incubated overnight in a CO₂ incubator. (Day 3) The sample was diluted using the medium, and 50 µL of the diluted sample, AGE-BSA in which AGE was formed with glyceraldehydes (50 µg/mL) as a control, and the mixture of diluted sample and AGE-BSA were added to wells. After incubation in a CO₂ incubator for 4 hours, the medium was removed and wells were washed 2 times with 300 µL of PBS (-) . Then, 25 µL of 1 x lysis buffer was added on ice. After the plate was shaken for 15 seconds, 20 µL of lysate was charged in a new white plate. Further 100 µL of a luciferase assay reagent was added, and a fluorescence intensity was measured by a luminometer. Using 5 µL of lysate, quantitative determination of protein was also performed.

### (3) Results

In the test section 3 containing sorbitol as a carbohydrate, the production of 3-DG was significantly decreased and the productions of fluorescent AGEs, pentosidine and CML were also decreased, compared with in the test sections 1 and 2 containing glucose and fructose as carbohydrates. In the test section 3 containing sorbitol as a carbohydrate, RAGE agonist activity was decreased and RAGE antagonist activity was also decreased, compared with in the test section 2 containing fructose as a carbohydrate. These results reveal AGEs and 3-DG production inhibitory effects of sorbitol in the presence of amino acid. The results of quantitative determination of various AGEs and 3-DG are shown in Table 11.

**Table 11**

| | 3-DG (nmol/1 mL of reaction solution) | Fluorescent AGEs (nmol/1 mL of reaction solution) | Pentosidine (nmol/1 mL of reaction solution) | CML (nmol/1 mL of reaction solution) | RAGE agonist activity* | RAGE antagonist activity* |
|---|---|---|---|---|---|---|
| Test section 1 (Amino acid + Glucose) | 8310 | 17.4 | 5.8 | 1197 | 0.06 | 0.56 |
| Test section 2 (Amino acid + Fructose) | 904 | 11.4 | 24.7 | 519 | 0.08 | 0.70 |
| Test section 3 (Amino acid + Sorbitol) | 13 | 0.4 | 0.8 | 57 | 0.07 | 0.66 |

| | | | | | | |
|---|---|---|---|---|---|---|
| *The values of the respective samples (for RAGE agonist activity), and the liquid mixture of the respective samples and AGE-BSA (for RAGE antagonist activity) relative to the value of control (AGE-BSA) were shown, assuming that the control value is 1. The value when the amount of protein is 100 µg/mL was shown. | | | | | | |

### Test Example 5

### (1) Preparation of samples

Biological proteins, carbohydrates and a phosphate buffer solution are charged in a 50 mL polypropylene centrifuge tube in each amount shown in Table 12, followed by sealing and stirring, and then the mixture was reacted in an incubator at 37°C for 42 days. Next, the reactant was fractionated by centrifugation at 3, 000 rpm for 3 hours using an ultrafiltration unit (VIVA SPIN 20, manufactured by Sartorius Corporation). The obtained low molecular weight fraction was used as a sample for determination of 3-DG, while the obtained high molecular weight fraction was used as a sample for the determination of fluorescent AGEs, pentosidine, CML, RAGE agonist activity and RAGE antagonist activity. Biological proteins and carbohydrates used are as follows:

### [Biological Proteins]

Bovine serum albumin (fatty acid free, low endotoxin, manufactured by Sigma-Aldrich Japan, LLC)
Human serum albumin (Fraction V, manufactured by Tokyo Chemical Industry Co., Ltd.)
γ-Globulin (derived from bovine blood, manufactured by Sigma-Aldrich Japan, LLC)

### [Carbohydrates]

Glucose (Special reagent grade, manufactured by Wako Pure Chemical Industries, Ltd.)
Fructose (KC, Reagent grade, manufactured by KATAYAMA
CHEMICAL, INC.)
Sorbitol (Sigma-Aldrich Japan, special grade, manufactured by Sigma-Aldrich Japan, LLC)

**Table 12**

| | Bovine serum albumin (g) | Human serum albumin (g) | γ-Globulin (g) | Glucose (g) | Fructose (g) | Sorbitol (g) | Phosphate buffer solution (mL) |
|---|---|---|---|---|---|---|---|
| Test section 1 | 0.4 | 0 | 0 | 1.8 | 0 | 0 | 20 |
| Test section 2 | 0.4 | 0 | 0 | 0 | 1.8 | 0 | 20 |
| Test section 3 | 0.4 | 0 | 0 | 0 | 0 | 1.8 | 20 |
| Test section 4 | 0 | 0.4 | 0 | 1.8 | 0 | 0 | 20 |
| Test section 5 | 0 | 0.4 | 0 | 0 | 1.8 | 0 | 20 |
| Test section 6 | 0 | 0.4 | 0 | 0 | 0 | 1.8 | 20 |
| Test section 7 | 0 | 0 | 0.4 | 1.8 | 0 | 0 | 20 |
| Test section 8 | 0 | 0 | 0.4 | 0 | 1.8 | 0 | 20 |
| Test section 9 | 0 | 0 | 0.4 | 0 | 0 | 1.8 | 20 |

### (2) Test Methods

In the same manner as in Test Example 4, except that the low molecular weight fraction prepared as mentioned above was used as a sample, quantitative determination of 3-DG was performed. In the same manner as in Test Example 4, except that the high molecular weight fraction was filtered through a 0.2 µm filter and the filtrate was used as a sample, fluorescent AGEs, pentosidine, CML, RAGE agonist activity and RAGE antagonist activity were evaluated.

### (3) Results

In the combination with each of the above biological proteins, in the test sections 3, 6 and 9 containing sorbitol as a carbohydrate, the productions of 3-DG, fluorescent AGEs, pentosidine and CML were decreased, and RAGE agonist activity and RAGE antagonist activity were also decreased, compared with in the test sections 1, 4 and 7 containing glucose as a carbohydrate. In the test sections 3, 6 and 9, the production of 3-DG was significantly decreased, the productions of fluorescent AGEs, pentosidine and CML were also decreased, and also RAGE agonist activity was decreased, compared with the test sections 2, 5 and 8 containing fructose as carbohydrate. In the test sections 3 and 6, RAGE antagonist activity was decreased. These results reveal AGEs and 3-DG production inhibitory effects of sorbitol in the presence of biological proteins. The results of quantitative determination of various AGEs and 3-DG are shown in Table 13.

**Table 13**

| | 3-DG (nmol/1 mL of reaction solution) | Fluorescent AGEs (nmol/1 mL of reaction solution) | Pentosidine (nmol/1 mL of reaction solution) | CML (nmol/1 mL of reaction solution) | RAGE agonist activity* | RAGE antagonist activity* |
|---|---|---|---|---|---|---|
| Test section 1 (Bovine serum albumin + Glucose) | 10253 | 11 | 0.01 | 309 | 0.37 | 1.08 |
| Test section 2 (Bovine serum albumin + Fructose) | 5316 | 28 | 0.03 | 1046 | 0.33 | 1.15 |
| Test section 3 (Bovine serum albumin + Sorbitol) | 33 | 2.0 | 0.03 | 10 | 0.24 | 1.08 |
| Test section 4 (Human serum albumin + Glucose) | 2575 | 8.6 | 0.02 | 1749 | 0.01 | 1.20 |
| Test section 5 (Human serum albumin + Fructose) | 2358 | 25.2 | 0.05 | 4626 | 0.02 | 1.33 |
| Test section 6 (Human serum albumin + Sorbitol) | 78 | 5.4 | 0.02 | 69 | 0.02 | 1.01 |
| Test section 7 (γ-Globulin + Glucose) | 877 | 3.0 | 0.002 | 3628 | 0.06 | 1.07 |
| Test section 8 (γ-Globulin + Fructose) | 877 | 17.3 | 0.005 | 3813 | 0.04 | 1.08 |
| Test section 9 (γ-Globulin + Sorbitol) | 21 | 1.3 | 0.002 | 29 | 0.06 | 0.90 |

| | | | | | | |
|---|---|---|---|---|---|---|
| *The values of the respective samples (for RAGE agonist activity), and the liquid mixture of the respective samples and AGE-BSA (for RAGE antagonist activity) relative to the value of control (AGE-BSA) was shown, assuming that the control value is 1. The value when the amount of protein is 100 µg/mL for bovine serum albumin and human serum albumin, and the value when the amount of protein is 10 µg/mL for γ-globulin were shown, respectively. | | | | | | |

### Test Example 6

### (1) Preparation of Samples

Collagen, carbohydrates and a phosphate buffer solution were charged in a 50 mL polypropylene centrifuge tube in each amount shown in Table 14, followed by sealing and stirring, and then the mixture was reacted in an incubator at 37°C for 42 days. Next, the reactant was fractionated by centrifugation at 3, 000 rpm for 3 hours using an ultrafiltration unit (VIVA SPIN 20, manufactured by Sartorius Corporation). The obtained low molecular weight fraction was used as a sample for determination of 3-DG. Meanwhile, 0.4 g of a high molecular weight fraction and 8 mL of 6N hydrochloric acid were charged in an ampule and the air in the ampule was replaced with nitrogen, and then hydrolysis was performed at 110°C for 16 hours in a heat block. After reaction, 8 mL of 5N sodium hydroxide was added, and pH was adjusted using 1N hydrochloric acid and 1N sodium hydroxide to make pH within a range of 7.2 to 7.6. Then, the solution was filtered through a 0.2 µm filter. The obtained filtrate was used as a sample for the measurement of fluorescent AGEs, pentosidine, CML, RAGE agonist activity and RAGE antagonist activity. The collagen and carbohydrates used are as follows.

### [Biological Proteins]

Collagen (Collagen Type I, derived from bovine dermis, pepsin solubilized, Nippi. Inc.)

### [Carbohydrates]

Glucose (Special reagent grade, manufactured by Wako Pure Chemical Industries, Ltd.)
Fructose (KC Special grade, manufactured by KATAYAMA CHEMICAL, INC.)
Sorbitol (Sigma-Aldrich Japan, Special grade, manufactured by Sigma-Aldrich Japan, LLC)

**Table 14**

| | Collagen (g) | Glucose (g) | Fructose (g) | Sorbitol (g) | Phosphate buffer solution (mL) |
|---|---|---|---|---|---|
| Test section 1 | 0.015 | 1.8 | 0 | 0 | 20 |
| Test section 2 | 0.015 | 0 | 1.8 | 0 | 20 |
| Test section 3 | 0.015 | 0 | 0 | 1.8 | 20 |

| | | | | | |
|---|---|---|---|---|---|
| *The amount of collagen in the table was expressed as the amount of protein when 5 mL of 3 mg/mL collagen is used. | | | | | |

### (2) Test Methods

In the same manner as in Test Example 4, except that the low molecular weight fraction prepared as mentioned above was used as a sample, quantitative determination of 3-DG was performed. In the same manner as in Test Example 4, except that the high molecular weight fraction filtrate prepared as mentioned above was used as a sample, fluorescent AGEs, pentosidine, CML, RAGE agonist activity and RAGE antagonist activity were evaluated.

### (3) Results

In the test section 3 containing sorbitol as a carbohydrate, the productions of 3-DG was significantly decreased, the productions of fluorescent AGEs, pentosidine and CML were decreased, and RAGE agonist activity and RAGE antagonist activity were decreased, compared with in the test sections 1 and 2 containing glucose and fructose as carbohydrates. The results of quantitative determination of various AGEs and 3-DG are shown in Table 15.

**Table 15**

| | 3-DG (nmol/1 mL of reaction solution) | Fluorescent AGEs (nmol/1 mL of reaction solution) | Pentosidine (nmol/1 mL of reaction solution) | CML (nmol/1 mL of reaction solution) | RAGE agonist activity* | RAGE antagonist activity* |
|---|---|---|---|---|---|---|
| Test section 1 (Collagen + Glucose) | 232 | 1.4 | 0.06 | 475 | 0.45 | 2.85 |
| Test section 2 (Collagen + Fructose) | 1447 | 2.4 | 0.15 | 452 | 0.58 | 2.49 |
| Test section 3 (Collagen + Sorbitol) | 24 | 0.5 | 0.05 | 445 | 0.27 | 1.22 |

| | | | | | | |
|---|---|---|---|---|---|---|
| *The values of the respective samples (for RAGE agonist activity) , and the liquid mixture of the respective samples and AGE-BSA (for RAGE antagonist activity) relative to the value of control (AGE-BSA) were shown, assuming that the control value is 1. The value when the amount of protein is 10 µg/mL was shown. | | | | | | |

### Formulation Example 1 (Powder)

Raw materials shown in Table 16 were mixed to produce powders. The powder is one embodiment of an AGEs production inhibitor or a 3-DG production inhibitor of the present invention, or of a medicament for the prevention/treatment of diabetes of the present invention.

**Table 16 (Formulation of powders)**

| Raw materials | Amount |
|---|---|
| Maltitol | 90g |
| Lactose | 10g |

### Formulation Example 2 (Tablets)

Raw materials shown in Table 17 were mixed and then direct compression was performed under tableting conditions of pestles (ϕ8 mm, R12 mm), weight per tablet of 150 to 200 mg, rotation speed of a rotary table of 12 rpm and a tableting pressure of 4 kN, using a continuous tableting press machine (Piccola B-10, manufactured by RIVA Ltd.) to produce tablets. The tablet is one embodiment of an AGEs production inhibitor or a 3-DG production inhibitor of the present invention, or of a medicament for the prevention/treatment of diabetes of the present invention.

**Table 17 (Formulation of tablets)**

| Raw materials | Amount |
|---|---|
| Maltitol | 94g |
| Dextrin | 5g |
| Calcium stearate | 1g |

### Formulation Example 3 (Liquids and Solutions for Oral Administration)

Raw materials shown in Table 18 were dissolved in 500 mL of distilled water to produce liquids and solutions for oral administration. The liquid and solution for oral administration is one embodiment of a medicament for the prevention/treatment of diabetes of the present invention.

**Table 18 (Formulation of liquids and solutions for oral administration)**

| Raw materials | Amount |
|---|---|
| Citric acid | 0.3g |
| Vimain B1 (Thiamine hydrochloride) | 0.05g |
| Vitamin C (L-ascorbic acid) | 0.06g |
| Sodium chloride | 0.1g |
| Isomerized sugar | 10g |
| Maltitol | 8g |

### Formulation Example 4 (Powders)

Raw materials shown in Table 19 were mixed to produce a powder. The powder is one embodiment of an AGEs production inhibitor or a 3-DG production inhibitor of the present invention, or of a medicament for the prevention/treatment of diabetes of the present invention.

**Table 19 (Formulation of powders)**

| Raw materials | Amount |
|---|---|
| Sorbitol | 90g |
| Lactose | 10g |

### Formulation Example 5 (Tablets)

Raw materials shown in Table 20 were mixed and then direct compression was performed under tableting conditions of pestles (ϕ8 mm, R12 mm), weight per tablet of 150 to 200 mg, rotation speed of a rotary table of 12 rpm and a tableting pressure of 4 kN, using a continuous tableting press machine (Piccola B-10, manufactured by RIVA Ltd.) to produce tablets. The tablet is one embodiment of an AGEs production inhibitor or a 3-DG production inhibitor of the present invention, or of a medicament for the prevention/treatment of diabetes of the present invention.

**Table 20 (Formulation of tablets)**

| Raw materials | Amount |
|---|---|
| Sorbitol | 94g |
| Dextrin | 5g |
| Calcium stearate | 1g |

### Formulation Example 6 (Liquids and Solutions for oral administration)

Raw materials shown in Table 21 were dissolved in 500 mL of distilled water to produce liquids and solutions for oral administration. The liquid and solution for oral administration is one embodiment of a medicament for the prevention/treatment of diabetes of the present invention.

**Table 21 (Formulation of liquids and solutions for oral administration)**

| Raw materials | Amount |
|---|---|
| Citric acid | 0.3g |
| Vitamin B1 (Thiamine hydrochloride) | 0.05g |
| Vitamin C (L-ascorbic acid) | 0.06g |
| Sodium chloride | 0.1g |
| Isomerized sugar | 10g |
| Sorbitol | 8g |

## Claims

1. A medicament comprising one or more sugar alcohols selected from maltitol and sorbitol as (a) sole active ingredient (s) for use in the prevention and/or treatment of diabetes or diabetic complications.

2. The medicament according to claim 1, which comprises 50% by weight or more of the sugar alcohol.

3. The medicament according to claim 1 or 2, wherein the prevention and/or treatment of diabetes or diabetic complications is/are achieved through the inhibition of the production of 3-deoxyglucosone or an advanced glycation end product.

4. The medicament according to any one of claims 1 to 3, wherein the sugar alcohol is maltitol.

5. The medicament according to any one of claims 1 to 3, wherein the sugar alcohol is sorbitol.

6. The medicament according to any one of claims 1-5, which is provided as a solution containing the sugar alcohol.

7. The medicament according to any one of claims 1-5, which is provided in the form of tablets, capsules, granules, powders, liquids and solutions for oral administration, syrups, jellies for oral administration, tablets for oro-mucosal application, sprays for oro-mucosal application, semi-solid preparations for oro-mucosal application, preparations for gargles, injections, dialysis agents, inhalations, suppositories for rectal application, semi-solid preparations for rectal application, enemas for rectal application, ophthalmic preparations, ophthalmic ointments, ear preparations, nasal preparations, tablets for vaginal use, suppositories for vaginal use, solid dosage forms for cutaneous application, liquids and solutions for cutaneous application, sprays for cutaneous application, ointments, creams, gels, or patches.

8. The medicament according to claim 7, which is provided in the form of powders.

9. The medicament according to claim 7, which is provided in the form of tablets.

10. The medicament according to claim 7, which is provided in the form of liquids and solutions for oral administration.

11. The medicament according to any one of claims 1-10, wherein diabetic complications are one or more diseases selected from the group consisting of diabetic retinopathy, diabetic nephropathy, diabetic neuropathy, diabetic vascular complication and arteriosclerosis.

## Patentansprüche

1. Medikament, welches einen oder mehrere Zuckeralkohol(e), ausgewählt aus Maltit und Sorbit, als (einen) einzige(n) Wirkstoff(e) zur Verwendung bei der Prävention und/oder Behandlung von Diabetes oder diabetischen Komplikationen umfasst.

2. Medikament gemäß Anspruch 1, welches 50 Gew.-% oder mehr von dem Zuckeralkohol umfasst.

3. Medikament gemäß Anspruch 1 oder 2, wobei die Prävention und/oder Behandlung von Diabetes oder diabetischen Komplikationen durch die Inhibition der Produktion von 3-Desoxyglucoson oder einem fortgeschrittenen Glykierunsendprodukt erzielt wird/werden.

4. Medikament gemäß einem der Ansprüche 1 bis 3, wobei der Zuckeralkohol Maltit ist.

5. Medikament gemäß einem der Ansprüche 1 bis 3, wobei der Zuckeralkohol Sorbit ist.

6. Medikament gemäß einem der Ansprüche 1-5, welches als eine Lösung bereitgestellt wird, welche den Zuckeralkohol beinhaltet.

7. Medikament gemäß einem der Ansprüche 1-5, welches in der Form von Tabletten, Kapseln, Granulaten, Pulvern, Flüssigkeiten und Lösungen zur oralen Verabreichung, Sirupe, Gelees zur oralen Verabreichung, Tabletten zur oromucosalen Anwendung, Sprays zur oromucosalen Anwendung, halbfesten Zubereitungen zur oromucosalen Anwendung, Zubereitungen für Mundwasser, Injektionen, Dialysemittel, Inhalationen, Zäpfchen zur rektalen Anwendung, halbfesten Zubereitungen zur rektalen Anwendung, Einläufe zur rektalen Anwendung, ophthalmischen Zubereitungen, ophthalmischen Salben, Ohrzubereitungen, nasalen Zubereitungen, Tabletten zur vaginalen Verwendung, Zäpfchen zur vaginalen Verwendung, festen Dosierformen zur kutanen Anwendung, Flüssigkeiten und Lösungen zur kutanen Anwendung, Sprays zur kutanen Anwendung, Salben, Cremes, Gelen oder Pflastern bereitgestellt wird.

8. Medikament gemäß Anspruch 7, welches in der Form von Pulvern bereitgestellt wird.

9. Medikament gemäß Anspruch 7, welches in Form von Tabletten bereitgestellt wird.

10. Medikament gemäß Anspruch 7, welches in Form von Flüssigkeiten und Lösungen zur oralen Verabreichung bereitgestellt wird.

11. Medikament gemäß einem der Ansprüche 1-10, wobei diabetische Komplikationen eine oder mehrere Krankheiten, ausgewählt aus der Gruppe, bestehend aus diabetischer Retinopathie, diabetischer Nephropathie, diabetischer Neuropathie, diabetischer vaskulärer Komplikation und Arteriosklerose, sind.

## Revendications

1. Médicament comprenant un ou plusieurs alcool(s) de sucre choisi (s) parmi le maltitol et le sorbitol en tant que seul ingrédient actif pour l'utilisation dans la prévention et/ou le traitement du diabète ou des complications diabétiques.

2. Médicament selon la revendication 1 qui comprend 50% en poids ou plus d'un alcool de sucre.

3. Médicament selon la revendication 1 ou 2, la prévention et/ou le traitement du diabète ou des complications diabétiques étant atteint(e) par l'inhibition de la production du 3-désoxy-glucosone ou d'un produit final de la glycation avancée.

4. Médicament selon l'une quelconque des revendications 1 à 3, dans lequel l'alcool de sucre est le maltitol.

5. Médicament selon l'une quelconque des revendications 1 à 3, dans lequel l'alcool de sucre est le sorbitol.

6. Médicament selon l'une quelconque des revendications 1 à 5, qui est fourni en tant que solution qui contient l'alcool de sucre.

7. Médicament selon l'une quelconque des revendications 1 à 5, qui est fourni sous forme de comprimés, de capsules, de granulat, de poudres, de liquides et de solutions pour l'administration orale, de comprimés pour l'application oro-muqueuse, de sprays pour l'application oro-muqueuse, de préparations demisolides pour l'application oro-muqueuse, de préparations pour gargarisme, d'injections, d'agents de dialyse, d'inhalations, de suppositoires pour l'application rectale, de clystères pour l'application rectale, de préparations ophtalmiques, de pommades ophtalmiques, de préparations pour les oreilles, de préparations nasales, de comprimés pour l'utilisation vaginale, de suppositoires pour l'utilisation vaginale, de formes posologiques solides pour l'application cutanée, de liquides et de solutions pour l'application cutanée, de sprays pour l'application cutanée, de pommades, de crèmes, de gels ou d'emplâtres.

8. Médicament selon la revendication 7, qui est fourni sous forme de poudres.

9. Médicament selon la revendication 7, qui est fourni sous forme de comprimés.

10. Médicament selon la revendication 7, qui est fourni sous forme de liquides ou de solutions pour l'administration orale.

11. Médicament selon l'une quelconque des revendications 1 à 10, les complications diabétiques étant une ou plusieurs maladie(s) choisie(s) dans le groupe constitué de la rétinopathie diabétique, la néphropathie diabétique, la neuropathie diabétique, d'une complication vasculaire du diabète et de l'artériosclérose.
